# EUROPEAN PATENT SPECIFICATION

(11) **EP 3 778 920 B1**
(45) Date of publication and mention of the grant of the patent: **07.02.2024**
(21) Application number: 19804006.5
(22) Date of filing: 28.03.2019
(51) Int. Cl.: C12Q 1/6806, C12Q 1/6844, C12Q 1/682

(54) **METHOD FOR THREE-DIMENSIONAL NUCLEIC ACID IMAGING DIAGNOSIS OF BIO-TISSUE BY USING ISOTHERMAL NUCLEIC ACID AMPLIFICATION**
VERFAHREN ZUR DREIDIMENSIONALEN NUKLEINSÄURE-BILDGEBUNGSDIAGNOSE VON BIOGEWEBE MITTELS ISOTHERMER NUKLEINSÄUREAMPLIFIKATION
PROCÉDÉ DE DIAGNOSTIC PAR IMAGERIE TRIDIMENSIONNELLE D'ACIDES NUCLÉIQUES D'UN BIO-TISSU, UTILISANT UNE AMPLIFICATION ISOTHERME D'ACIDES NUCLÉIQUES

(30) Priority: 18.05.2018 KR 20180057465
(43) Date of publication of application: 17.02.2021
(73) Proprietor: Binaree, Inc., Daegu 41566 (KR)
(72) Inventor: PARK, Young Il, Daegu 42014 (KR); GUM, Sang Il, Gyeongsangbuk-do 37655 (KR); JEON, Hyo Sung, Daegu 42062 (KR)
(74) Representative: Dehns
(86) International application number: PCT/KR2019/003622
(87) International publication number: WO 2019/221384

(56) References cited:
- WO-A1-2017/031249
- WO-A1-2017/139501
- CN-A- 107 621 462
- JP-A- 2015 096 061
- KR-A- 20170 022 854
- KR-B1- 101 849 704
- KR-B1- 101 849 704
- US-A1- 2016 265 046
- MARUYAMA, F. et al.: "Detection of bacteria carrying the stx2 gene by in situ loop-mediated isothermal amplification", Applied and Environmental Microbiology, vol. 69, no. 8, 2003, pages 5023-5028, XP003012385, DOI: 10.1128/AEM.69.8.5023-5028.2003
- DENG, R. et al.: "Isothermal amplification for microRNA detection: from the test tube to the cell", Accounts of Chemical Research, vol. 50, 29 March 2017 (2017-03-29), pages 1059-1068, XP055653636, DOI: 10.1021/acs.accounts.7b00040
- HAMA, H. et al.: "Scale: a chemical approach for fluorescence imaging and reconstruction of transparent mouse brain", Nature Neuroscience, vol. 14, no. 11, November 2011 (2011-11), pages 1481-1488, XP055507756, DOI: 10.1038/nn.2928
- KUMAR, Y. et al.: "Loop-Mediated Isothermal Amplification (LAMP): A rapid and sensitive tool for quality assessment of meat products", Comprehensive Reviews in Food Science and Food Safety, vol. 16, November 2017 (2017-11), pages 1359-1378, XP055752536, DOI: 10.1111/1541-4337.12309

## Description

### [Technical Field]

The present invention relates to a method for three-dimensional nucleic acid imaging diagnosis of tissue by using isothermal nucleic acid amplification.

### [Background Art]

Among test methods used in diagnosis of a disease, immunochemical or imaging diagnosis using a protein has difficulty in diagnosing with a small amount of biomarkers because there are low specific responses to an antibody, and the biomarkers cannot be amplified.

Therefore, to overcome the above problems, for diagnosis of various diseases including cancer, research on a molecular biomarker detecting a disease with DNA and RNA that carries genetic information in the human body is being widely conducted. DNA and RNA biomarkers are used in diagnosis of various diseases through methods such as PCR, microchips and NGS, and in this case, polymerase chain reaction (PCR) is the most widely used technique among nucleic acid amplification techniques which detect and analyze a small amount of nucleic acids, and a method performed by repeating steps of denaturing double-stranded DNA into single-stranded DNA at high temperature, binding primers to the single strand at a lower temperature, and performing extension into double-stranded DNA using Taq polymerase (thermostable enzyme). However, the DNA and RNA biomarkers have a disadvantage in that cells expressed in tissue and locations thereof cannot be known.

To solve this problem, the presence and location of DNA and RNA biomarkers in tissue may be known by a hybridization method such as FISH, but this method has difficulty in diagnosis because this method cannot amplify DNA or RNA, as well as having a limit to observation due to low tissue permeability.

Meanwhile, loop-mediated isothermal amplification (LAMP) is a detection method invented by Notomi *et al.* in 2000, which is similar to conventional PCR, but does not need temperature changes required for DNA denaturation, primer annealing and extension of a polymerase in PCR or real-time PCR and enables amplification at a temperature close to 60 °C.

The biggest characteristic of LAMP is isothermal amplification of a gene, and due to no need of temperature control, compared to PCR requiring a temperature gradient, LAMP has a relatively short reaction time. Thus, this method enables gene amplification within a shorter time, and by using LAMP, it was previously reported that gene amplification is possible within one hour excluding electrophoresis time. In addition, since there are no DNA loss and damage according to a temperature change, amplification efficiency is very high, and amplification under an isothermal condition indicates that LAMP can have practicability compared to other detection methods. Since LAMP only needs maintenance of a constant temperature, it does not require expensive equipment, and facilitates a reaction only with simple equipment such as a water bath. Therefore, according to LAMP, even when not in a laboratory environment, detection of a specific gene is possible in practice.

However, related research on a diagnosis method using an isothermal amplification method is not sufficient yet.

WO2017139501 relates to RNA fixation and detection in clarity-based hydrogel tissue.

### [Disclosure]

### [Technical Problem]

As a result of earnest attempts to solve the above-described problems of a diagnosis method using a biomarker, a method for three-dimensional nucleic acid imaging diagnosis of tissue, which may improve diagnostic accuracy by clearing tissue through light transmission to clearly see a specific molecular biomarker in tissue, and facilitate three-dimensional imaging of a biomarker using an isothermal amplification method in tissue, as defined in present claim 1, was invented, and thus the present invention was completed.

### [Technical Solution]

To achieve the above-described object, a method as defined in present claim 1, for three-dimensional nucleic acid imaging diagnosis of tissue, which comprises: (a) clearing a tissue sample;
(b) isothermally amplifying a biomarker to be detected by adding an enzyme reaction mixed solution and a primer to the cleared tissue sample obtained in Step (a); and
(c) detecting the biomarker amplified in Step (b), is provided.

In one embodiment of the present invention, the primer used in Step (b) may be a primer of a biomarker to be amplified.

In another embodiment of the present invention, the primer may comprise a probe.

In still another embodiment of the present invention, the tissue may be a brain, a liver, a lung, a kidney, an intestine, a heart, a muscle or a blood vessel.

According to the present invention, the clearing process in Step (a) comprises
(i) fixing a tissue sample by adding it to a fixing solution;
(ii) reacting the fixed sample in a tissue clearing solution; and
(iii) adding the sample reacted in the tissue clearing solution to a washing solution to wash an organic material attached to the sample.

According to the present invention, the fixing solution comprises sucrose.

In yet another embodiment of the present invention, a concentration of the sucrose may be 20 to 100%(w/v).

According to the present invention, the tissue clearing solution comprises 3-[(3-cholamidopropyl)dimethylammonio]-1-propanesulfonate (CHAPS), urea and sodium chloride (NaCl).

In yet another embodiment of the present invention, a concentration of the NaCl may be 0.001 to 1.0%(w/v).

According to the present invention, the washing solution comprises phosphate buffer saline (PBS) and sodium azide.

In yet another embodiment of the present invention, a concentration of the sodium azide may be 0.001 to 0.5%(w/v).

In yet another embodiment of the present invention, the biomarker may be a molecular biomarker.

In yet another embodiment of the present invention, the molecular biomarker may be DNA or RNA.

### [Advantageous Effects]

A method for three-dimensional nucleic acid imaging diagnosis of tissue using isothermal nucleic acid amplification according to the present invention can allow a specific molecular biomarker to be clearly seen in tissue through clearing of the tissue, enhance diagnostic accuracy by three-dimensionally reconstituting the tissue since all of the inside of the tissue is visualized, and facilitate three-dimensional imaging of a molecular biomarker such as DNA or RNA containing genetic information in the human body through isothermal nucleic acid amplification in tissue, and thus this method is expected to be effectively used in diagnosis of various diseases including cancer.

### [Description of Drawings]

FIG. 1 is a view showing a result of isothermal nucleic acid amplification of Thy-1 mRNA in mouse brain tissue according to one embodiment of the present invention.
FIG. 2 is a view showing a result of isothermal nucleic acid amplification of GAD-67 mRNA in mouse brain tissue according to one embodiment of the present invention.

### [Modes of the Invention]

Hereinafter, the present invention will be described in detail.

The present invention provides a method as defined in present claim 1 for three-dimensional nucleic acid imaging diagnosis of tissue, which comprises: (a) clearing a tissue sample;
(b) isothermally amplifying a biomarker to be detected by adding an enzyme reaction mixed solution and a primer to the cleared tissue sample obtained in Step (a); and
(c) detecting the biomarker amplified in Step (b).

The primer used in Step (b) may be a primer of a biomarker to be amplified, and comprise a probe.

The tissue in Step (a) may be a brain, a liver, a lung, a kidney, an intestine, a heart, a muscle or a blood vessel, but the present invention is not limited thereto.

In addition, the clearing process in Step (a) comprises: (i) fixing a tissue sample by adding it to a fixing solution;
(ii) reacting the fixed sample in a tissue clearing solution; and
(iii) adding the sample reacted in the tissue clearing solution to a washing solution to wash an organic material attached to the sample.

The fixing solution comprises sucrose, and here, a concentration of the sucrose may be 20 to 100%(w/v). According to one embodiment of the present invention, the sucrose concentration may be 20 to 80%(w/v), 20 to 60%(w/v), 20 to 40%(w/v), 20 to 30%(w/v), 60 to 100%(w/v), or 80 to 100%(w/v), but when the sucrose concentration is 20%(w/v) or more, the sucrose concentration is not limited thereto.

The tissue clearing solution comprises 3-[(3-cholamidopropyl)dimethylammonio]-1-propanesulfonate (CHAPS), urea and sodium chloride (NaCl), and here, a concentration of the NaCl may be 0.001 to 1.0%(w/v), and according to one embodiment of the present invention, the NaCl concentration may be 0.01 to 0.7%(w/v), 0.01 to 0.5%(w/v), 0.1 to 0.7%(w/v), 0.1 to 0.5%(w/v), 0.1 to 0.3%(w/v), or 0.3 to 0.5%(w/v), but the NaCl concentration is not limited thereto.

The washing solution comprises phosphate buffer saline (PBS) and sodium azide. Here, a concentration of the sodium azide may be 0.001 to 0.5%(w/v), and according to one embodiment of the present invention, the sodium azide concentration may be 0.01 to 0.4%(w/v), 0.01 to 0.3%(w/v), 0.01 to 0.2%(w/v), or 0.1%(w/v), but the sodium azide concentration is not limited thereto.

The "biomarker" in Step (b) is molecular information derived from DNA, RNA, a metabolite, a protein or a protein fragment, and an indicator that can detect changes in a human body caused by the onset of a disease. The biomarker is associated with the onset and progression of a disease, and thus is widely used in development of a novel drug, and development of *in vitro* molecular diagnosis technique for early detection of a disease *in vitro* and observing its prognosis, a personalized medical technique for identifying personalized characteristics of a biomarker responding to a specific drug, and a ubiquitous healthcare system to establish a patient-friendly treatment environment. Since appearing differently in a living organism depending on the type of a disease and the onset and progression thereof, the biomarker is a material serving as an indicator in blood or a body fluid that can objectively detect a specific disease or drug reaction state, and serves to determine a disease early by only analyzing the blood or body fluid.

In the present invention, the biomarker may be a molecular biomarker, and specifically DNA or RNA, but the present invention is not limited thereto.

In one embodiment of the present invention, after clearing the tissue sample (see Example 1), three-dimensional nucleic acid images of tissue were confirmed using isothermal nucleic acid amplification (see Example 2).

### [Examples]

Hereinafter, to help in understanding the present invention, exemplary examples will be suggested. However, the following examples are merely provided to more easily understand the present invention, and not to limit the present invention.

### Example 1. Clearing of tissue sample

A brain was removed from a mouse and cut into 3-mm fragments, and then the fragmented brain samples were added to a fixing solution and reacted at 4 °C for 12 hours.

Afterwards, the samples were reacted in a tissue clearing solution at 37 °C for 6 hours.

The samples reacted in the tissue clearing solution were added to a washing solution and reacted at room temperature for 6 hours for clearing.

Components of the solutions used for clearing the tissue samples are shown in Table 1 below.

**[Table 1]**

| | Component |
|---|---|
| Fixing solution | Sucrose (20%(w/v) or more) |
| Tissue clearing solution | CHAPS (20%(w/v)), Urea (50%(w/v)), NaCl (0.1 to 0.5%(w/v)) |
| Washing solution | PBS, sodium azide (0.1%(w/v)) |

### Example 2. Confirmation of three-dimensional nucleic acid images of tissue using isothermal nucleic acid amplification

### 2-1. Confirmation of result of Thy-1 mRNA isothermal nucleic acid amplification in mouse brain

An enzyme reaction mixed solution containing Bst DNA polymerase and reverse transcriptase was added to the mouse brain samples cleared in Example 1 and shaken at 4 °C for 24 hours, and to determine whether isothermal amplification was possible in tissue, as a marker for neurons, thymocytes, T cells and stem cells, thymocyte differentiation antigen 1 (Thy-1) mRNA highly expressed in a mouse brain tissue was isothermally amplified.

Specifically, a Thy1 primer (including a probe) was added to the mouse brain sample and shaken at 4 °C for 12 hours, and then washed with 2x buffer at room temperature for 6 hours. The Thy1 primer (including a probe) used herein is shown in Table 2 below.

After washing, in a reaction buffer such as a 2x buffer containing deoxynucleoside triphosphates (dNTPs), the samples were reacted at 4 °C for 1 hour, and then incubated at 58 °C for 5 to 20 minutes.

After incubation, the samples were reacted in a washing solution at room temperature for 6 hours, and reacted in a mounting solution at 37 °C for 6 hours, followed by detection of Thy-1 mRNA using light sheet microscopy.

Components of the solutions used for the isothermal nucleic acid amplification of Thy-1 mRNA are shown in Table 3 below.

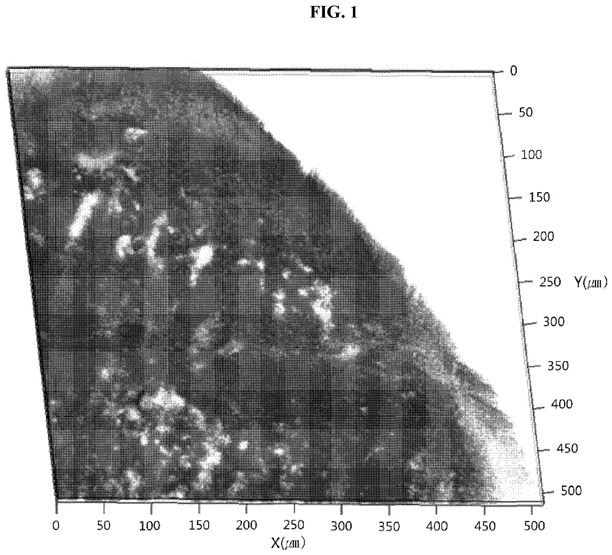

As a result, as shown in FIG. 1, by performing isothermal nucleic acid amplification of Thy-1 mRNA, it was able to be confirmed that a location of the Thy-1 mRNA in the mouse brain is clearly shown by green fluorescence.

**[Table 2]**

| **Oligo name** | Primer | Sequence | SEQ ID NO: |
|---|---|---|---|
| Thy1-A | F3 | GGG AGT CCA GAA TCC AAG | 1 |
| Thy1-B | B3 | CGT GTG CTC GGG TAT C | 2 |
| Thy1-C | FIP | TGG TCA CCT TCT GCC CTC CTT GGC ACC ATG AAC CC | 3 |
| Thy1-D | BIP | CTT CGC CTG GAC TGC C TGC TTC CTC TTC TCT CGG | 4 |
| Thy1-E | LF | CAA GAC TGA GAG CAG GAG AGC G | 5 |
| Thy1-F | LB | TCC ATC CAG CAT GAG TTC AGC C | 6 |
| Thy1-HEX | Fluorescent | CAC CAC CCT CCC GTG GGC GGC AAG ACT GAG AGC AGG AGA GCG | 7 |
| Thy-1-BHQ1 | Quencher | CCG CCC ACG GGA GGG TGG TG | 8 |

**[Table 3]**

| | Component |
|---|---|
| 2x buffer (-dNTP) | 20 mM Tris-HCl (pH 8.8), 10 mM KCl, 4 mM MgSO₄, 10 mM(NH₄)₂SO₄, 0.1% Triton X-100 |
| 2x reaction buffer | 20 mM Tris-HCl (pH 8.8), 10 mM KCl, 4 mM MgSO₄, 10 mM (NH₄)₂SO₄, 0.1% TritonX-100, 1.6 mM dNTPs |
| Washing solution | PBS, sodium azide (0.1%(w/v)) |
| Mounting solution | CHAPS (40%(w/v)), Urea (30%(w/v)), NaCl (0.1 to 1%(w/v)) |

### 2-2. Confirmation of result of isothermal nucleic acid amplification of GAD-67 mRNA in mouse brain

By the same method as the method described in Example 2-1, glutamic acid decarboxylase 67 (GAD-67) mRNA of a mouse brain sample was isothermally amplified. A GAD67 primer (including a probe) used herein is shown in Table 4 below.

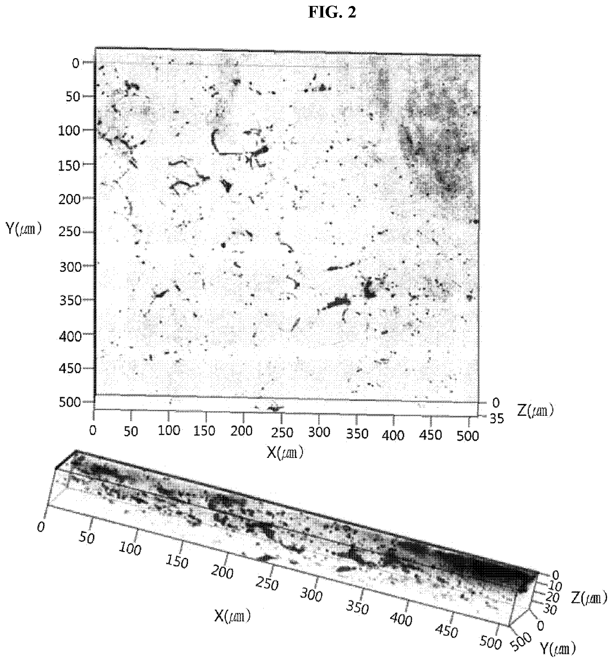

As a result, as shown in FIG. 2, it was able to be confirmed that, by isothermal nucleic acid amplification of GAD-67 mRNA, a location of GAD-67 mRNA in the mouse brain is clearly shown in red.

**[Table 4]**

| **Oligo name** | Primer | Sequence | SEQ ID NO: |
|---|---|---|---|
| gad67-A | F3 | GCA AGA CAT TTG ATC GCT CC | 9 |
| gad67-B | B3 | GAG AAC AAA CAC GGG TGC | 10 |
| gad67-C | FIP | TGC TCC AGA GAC TCG GGG ATT TCC ACCACC CAC ACC | 11 |
| gad67-D | BIP | CGC ACA GGT CAC CCT CG ATG TCA GCC ATT CAC CAG C | 12 |
| gad67-E | LF | GCC TTC CAT GCC TTC CAG | 13 |
| gad67-F | LB | ACC AGC TCT CTA CTG GTT TGG | 14 |
| gad67-Cy5 | Fluorescent | GCC ACA GCC CTC TCC CGC CGG CCT TCC ATG CCT TCC AG | 15 |
| gad67-BHQ3 | Quencher | CGGCGG GAG AGG GCT GTG GC | 16 |

From the result of the above example, by using the method for three-dimensional nucleic acid imaging diagnosis according to the present invention, it was confirmed that accurate diagnosis performed by confirming a location of a biomarker in tissue through tissue clearing and isothermal amplification is possible.

It should be understood by those of ordinary skill in the art that the above descriptions of the present invention are exemplary, and the example embodiments disclosed herein can be easily modified into other specific forms.

Therefore, it should be interpreted that the example embodiments described above are exemplary in all aspects, and are not limitative. The present invention is defined by the appended claims.

### [Industrial Applicability]

It is expected that a method for three-dimensional nucleic acid imaging diagnosis of tissue using isothermal nucleic acid amplification according to the present invention is effectively used to diagnose various diseases including cancer by facilitating three-dimensional imaging of a molecular biomarker such as DNA or RNA containing genetic information in the human body through isothermal nucleic acid amplification in tissue.

## Claims

1. A method for three-dimensional nucleic acid imaging diagnosis of tissue, comprising:
(a) clearing a tissue sample, wherein the clearing process comprises:
(i) fixing a tissue sample by adding it to a fixing solution comprising sucrose,
(ii) reacting the fixed sample in a tissue clearing solution comprising 3-[(3-cholamidopropyl)dimethylammonio]-1-propanesulfonate (CHAPS), urea and sodium chloride (NaCl), and
(iii) adding the sample reacted in the tissue clearing solution to a washing solution comprising phosphate buffer saline (PBS) and sodium azide to wash an organic material attached to the sample;
(b) isothermally amplifying a biomarker to be detected by adding an enzyme reaction mixed solution and a primer to the cleared tissue sample obtained in Step (a); and
(c) detecting the biomarker amplified in Step (b).

2. The method of claim 1, wherein the primer used in Step (b) is a primer of a biomarker to be amplified.

3. The method of claim 1 or claim 2, wherein the primer comprises a probe.

4. The method of any one of claims 1 to 3, wherein the tissue is a brain, a liver, a lung, a kidney, an intestine, a heart, a muscle or a blood vessel.

5. The method of any one of claims 1 to 4, wherein a concentration of the sucrose is 20 to 100%(w/v).

6. The method of any one of claims 1 to 5, wherein a concentration of the sodium chloride (NaCl) is 0.001 to 1.0%(w/v).

7. The method of any one of claims 1 to 6, wherein a concentration of the sodium azide is 0.001 to 0.5%(w/v).

8. The method of any one of claims 1 to 7, wherein the biomarker is a molecular biomarker.

9. The method of claim 8, wherein the molecular biomarker is DNA or RNA.

## Patentansprüche

1. Verfahren zur dreidimensionalen Nukleinsäure-Bildgebungsdiagnose von Gewebe, umfassend:
(a) Reinigen einer Gewebeprobe, wobei der Reinigungsprozess Folgendes umfasst:
(i) Fixieren einer Gewebeprobe durch ihre Zugabe zu einer Saccharose enthaltenden Fixierlösung,
(ii) Umsetzen der fixierten Probe in einer Gewebereinigungslösung, die 3-[(3-Cholamidopropyl)dimethylammonio]-1-propansulfonat (CHAPS), Harnstoff und Natriumchlorid (NaCl) enthält, und
(iii) Zugabe der in der Gewebereinigungslösung umgesetzten Probe zu einer Waschlösung, die Phosphatpuffersalzlösung (PBS) und Natriumazid enthält, um ein an die Probe gebundenes organisches Material zu waschen;
(b) isothermisches Verstärken eines zu erfassenden Biomarkers durch Zugabe einer gemischten Enzymreaktionslösung und eines Primers zu der in Schritt (a) erhaltenen gereinigten Gewebeprobe; und
(c) Erfassen des verstärkten Biomarkers in Schritt (b).

2. Verfahren nach Anspruch 1, wobei der Primer, der in Schritt (b) verwendet wird, ein Primer eines zu verstärkenden Biomarkers ist.

3. Verfahren nach Anspruch 1 oder Anspruch 2, wobei der Primer eine Sonde umfasst.

4. Verfahren nach einem der Ansprüche 1 bis 3, wobei das Gewebe ein Gehirn, eine Leber, eine Lunge, eine Niere, ein Darm, ein Herz, ein Muskel oder ein Blutgefäß ist.

5. Verfahren nach einem der Ansprüche 1 bis 4, wobei eine Konzentration der Saccharose 20 bis 100 % (w/v) beträgt.

6. Verfahren nach einem der Ansprüche 1 bis 5, wobei eine Konzentration des Natriumchlorids (NaCl) 0,001 bis 1,0 % (w/v) beträgt.

7. Verfahren nach einem der Ansprüche 1 bis 6, wobei eine Konzentration des Natriumazids 0,001 bis 0,5 % (w/v) beträgt.

8. Verfahren nach einem der Ansprüche 1 bis 7, wobei der Biomarker ein molekularer Biomarker ist.

9. Verfahren nach Anspruch 8, wobei der molekulare Biomarker DNA oder RNA ist.

## Revendications

1. Procédé de diagnostic par imagerie tridimensionnelle d'acides nucléiques d'un tissu, comprenant :
(a) la clarification d'un échantillon de tissu, dans lequel le processus de nettoyage comprend :
(i) la fixation d'un échantillon de tissu en l'ajoutant à une solution de fixation comprenant du saccharose,
(ii) la mise en réaction de l'échantillon fixé dans une solution de clarification de tissu comprenant du 3-[(3-cholamidopropyl)diméthylammonio]-1-propanesulfonate (CHAPS), de l'urée et du chlorure de sodium (NaCl), et
(iii) l'ajout de l'échantillon ayant réagi dans la solution de clarification de tissu à une solution de lavage comprenant une solution saline de tampon phosphate (PBS) et de l'azoture de sodium pour laver une matière organique attachée à l'échantillon ;
(b) l'amplification isotherme d'un biomarqueur à détecter par l'ajout d'une solution mixte de réaction enzymatique et d'une amorce à l'échantillon de tissu clarifié obtenu à l'étape (a) ; et
(c) la détection du biomarqueur amplifié à l'étape (b).

2. Procédé selon la revendication 1, dans lequel l'amorce utilisée à l'étape (b) est une amorce d'un biomarqueur à amplifier.

3. Procédé selon la revendication 1 ou la revendication 2, dans lequel l'amorce comprend une sonde.

4. Procédé selon l'une quelconque des revendications 1 à 3, dans lequel le tissu est un cerveau, un foie, un poumon, un rein, un intestin, un coeur, un muscle ou un vaisseau sanguin.

5. Procédé selon l'une quelconque des revendications 1 à 4, dans lequel la concentration de saccharose est comprise entre 20 et 100 % (p/v).

6. Procédé selon l'une quelconque des revendications 1 à 5, dans lequel la concentration de chlorure de sodium (NaCl) est comprise entre 0,001 et 1,0 % (p/v).

7. Procédé selon l'une quelconque des revendications 1 à 6, dans lequel la concentration d'azoture de sodium est comprise entre 0,001 et 0,5 % (p/v).

8. Procédé selon l'une quelconque des revendications 1 à 7, dans lequel le biomarqueur est un biomarqueur moléculaire.

9. Procédé selon la revendication 8, dans lequel le biomarqueur moléculaire est l'ADN ou l'ARN.
